# EUROPÄISCHE PATENTSCHRIFT

(11) **EP 0 437 004 B1**
(45) Veröffentlichungstag und Bekanntmachung des Hinweises auf die Patenterteilung: **19.07.1995**
(21) Anmeldenummer: 90250282.2
(22) Anmeldetag: 15.11.1990
(51) Int. Cl.: A61B 19/00

(54) **Folienüberzug zum Schutz eines chirurgischen Instrumentes**
Foil cover for the protection of a surgical instrument
Film de protection pour un appareil chirurgical

(30) Priorität: 16.11.1989 DE 8913630 U; 29.11.1989 DE 8914215 U
(43) Veröffentlichungstag der Anmeldung: 17.07.1991
(73) Patentinhaber: Effner Biomet GmbH, D-12247 Berlin (DE)
(72) Erfinder: Anapliotis, Emmanuel, W-1000 Berlin 33 (DE)
(74) Vertreter: Christiansen, Henning, Dipl.-Ing.

(56) Entgegenhaltungen:
- GB-A- 163 146
- GB-A- 2 148 526
- US-A- 4 413 278
- US-A- 4 522 196
- US-A- 4 722 000
- US-A- 4 736 733
- US-A- 4 756 304
- US-A- 4 834 068
- US-A- 4 844 071

## Beschreibung

Die Erfindung betrifft einen Folienüberzug der im Oberbegriff des Anspruchs 1 angegebenen Art.

Optische Sehhilfen für medizinische Anwendungen sind zunehmend nur noch für Bildschirmbeobachtung bzw. Dokumentation des jeweiligen Operationsablaufes konzipiert. Die herkömmliche Okularbeobachtung ist aufgrund optischer und ergonomischer Nachteile hauptsächlich auf stereoskopische Strahlengänge und wenig zeitaufwendige Anwendungsfälle beschränkt.

Die modernen Kameraendoskope, spezielle Geräte zur Verwendung mit Foto-, Film- oder Videokameras, sind im allgemeinen sehr kompakt gebaut. Um die wertvollen Kameras zu schützen und häufiges Sterilisieren der Kameras zu vermeiden, werden diese einschließlich der Video- und/oder anderer Kabel durch Folienüberzüge von der Operationswunde abgeschirmt.

Ein aus der DE-GM 88 12 027 bekannter Folienüberzug besteht aus einem beidseitig geöffneten Folienschlauch, wobei der Folienschlauch vor der Benutzung im allgemeinen teleskopartig im Innenraum eines ringförmigen Behälters gefaltet ist. Nach dem Herausziehen eines Endes des Folienschlauches aus dem Behälter wird die unsterile Kamera von der einen und das Arthroskop durch die ringförmige Öffnung des Behälters von der anderen Seite in den Folienschlauch eingeführt und eine steril abdichtende Verbindung zwischen dem Arthroskop und dem Folienschlauch wird mittels Schlauchbinder hergestellt. Danach wird das andere Ende des Folienschlauches aus dem Behälter und über den die Kamera umhüllenden Schlauchfolienabschnitt gezogen und ebenfalls mit einem Schlauchbinder verschlossen. Die Handhabung eines derartigen Folienüberzuges ist, insbesondere wegen der Schlauchbinder, relativ zeitaufwendig und erfordert einige Geschicklichkeit und ist zudem in bezug auf die Dichtheit der Verbindung kaum reproduzierbar. Die Schlauchbinder können unter Umständen die Bedienung des Instrumentes behindern. Darüberhinaus muß zwischen zwei Operationen oder auch im Verlaufe einer Operation, sobald ein Austausch des Endoskops bzw. der Endoskopoptik erforderlich ist, ein neuer Folienüberzug angelegt werden, da die Verbindungsstelle zwischen dem Endoskop und der Kamera innerhalb des Folienüberzuges liegt.

Bei einem weiteren aus der DE-GM 87 11 189 bekannten Folienüberzug besteht dieser aus einem Folienschlauch mit einer Einsteck- und einer Ausgangsöffnung, wobei der Schlauchteil vor der Benutzung im allgemeinen teleskopartig gefaltet ist. Die Faltung erleichtert einerseits das Einschieben eines Instrumentes, beispielsweise einer Kamera, welches ein Kupplungselement aufweist und dient andererseits der bedarfsweisen Längenanpassung des Folienüberzuges an das jeweilige Instrument. Die Ausgangsöffnung weist eine als Anschlag für das einzuschiebende Instrument wirkende Schlauchverengung auf. Beim Verbinden der Kamera mit dem Arthroskop faltet sich der Folienschlauch zwischen die Anlageflächen der Kupplungselemente der Kamera und des Arthroskops, da die Schlauchverengung derart dimensioniert ist, daß deren Durchmesser kleiner ist als der Durchmesser der Kupplungselemente des Arthroskops. Nachteilig hierbei ist, daß die durch eine Schlauchverengung gebildete Ausgangsöffnung dadurch möglichst genau an den Querschnitt des einzuschiebenden Instrumententeils angepaßt sein muß, so daß die Anzahl der benötigten Konfektionsvarianten der Folienüberzüge von den Querschnitten der angewendeten Instrumente abhängt.

Ein weiterer, aus US-A-4 522 196 bekannter Folienüberzug der gattungsgemäßen Art weist spezielle Befestigungsmittel zur Befestigung an der Objektiv-Endoskop-Verbindungsstelle eines speziellen Arthroskopes auf, die dort mit dem Arthroskop in Eingriff gebracht werden. Diese - in der Druckschrift in verschiedenen Ausführungen beschriebenen - Befestigungsmittel sind relativ kostenaufwendige und vergleichsweise umständlich zu handhabende Präzisionsteile, die zudem nur zu einem speziellen Arthroskop passen. Der beschriebene Folienüberzug ist damit insgesamt nicht kostengünstig herstellbar und in einer Ausführung für verschiedene Instrumente verwendbar.

Der Erfindung liegt die Aufgabe zugrunde, bei einem Folienüberzug der eingangs genannten Gattung die Abdichtung der Ausgangsöffnung zu verbessern, wobei gleichzeitig eine Vereinfachung der Handhabbarkeit und eine Verringerung der benötigten Konfektionsvarianten des Folienüberzuges anzustreben ist.

Diese Aufgabe wird mit den kennzeichnenden Merkmalen des Anspruchs 1 gelöst.

Die Erfindung beruht auf der Erkenntnis, daß sowohl Schlauchbinder oder sonstige Klebe- oder Schleifenbänder als auch eine Schlauchverengung zur dichten Verbindung des sterilen mit dem mit einem sterilen Folienüberzug abgedeckten Teil des nicht sterilen Instruments durch eine mit einer Durchbrechung versehenen elastischen Membran, deren äußerer Rand in einem den Folienschlauch aufspreizenden Rahmen eingespannt ist und die sich dicht an das Instrumententeil anschmiegt, ersetzbar ist, wodurch die Dichtheit der Ausgangsöffnung und die Handhabbarkeit des gesamten Folienüberzuges verbessert werden.

Besonders vorteilhaft bei dem erfindungsgemäßen Folienüberzug ist die gute Anpassung der elastischen Membran an unterschiedliche Querschnittsformen und Größen des hindurchragenden Teiles des chirurgischen Instrumentes. Bei entsprechend hoher Elastizität der Membran läßt sich somit eine Membran mit einer Durchbrechung einer bestimmten Größe und damit eine Ausführungsform des Folienüberzuges für verschiedene Anwendungsfälle nutzen, so daß die Anzahl der Varianten reduzierbar ist.

Die Membran ist vorzugsweise kreisförmig ausgebildet und mit einer zentrischen, ebenfalls kreisförmigen Durchbrechung versehen. Dementsprechend weist der Rahmen, mit welchem sowohl der Folienschlauchrand als auch die Membran verbunden ist, ringförmige bzw. kreiszylinderförmige Konturen auf.

Die Verbindung zwischen dem Rahmen und dem Folienschlauchrand ist entsprechend einer vorteilhaften Ausführungsform der Erfindung als Schweißverbindung zwischen einem ringförmigen, schweißbaren Ansatz, insbesondere Weichplastikansatz, des Rahmens und dem Rand des Folienschlauches ausgebildet.

Gemäß einer anderen vorteilhaften Ausführungsform besteht der Rahmen aus mehreren Profilringen, zwischen denen der Folienschlauchrand und/oder die Membran eingespannt sind. Die Profilringe sind dabei bevorzugt durch Klemmsitz oder Verschraubung miteinander verbunden.

Der Rahmen besteht vorzugsweise aus Kunststoff, während für die Membran Gummi-, Silikon- oder Latexmaterial besonders geeignet sind.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine perspektivische Ansicht eines erfindungsgemäßen Folienüberzuges,
Figur 2 eine Schnittdarstellung einer ersten Ausführungsform einer Ausgangsöffnung als Detail des Folienüberzuges gemäß Figur 1,
Figur 3 eine Schnittdarstellung einer zweiten Ausführungsform einer Ausgangsöffnung als Detail des Folienüberzuges gemäß Figur 1,
Figur 4 eine Prinzipdarstellung eines als Arthroskop ausgebildeten Endoskops mit einer Kamera und einem erfindungsgemäßen Folienüberzug gemäß Figur 1,
Figur 5 eine Schnittdarstellung eines Arthroskops gemäß Figur 4 mit einer ersten Ausführungsvariante einer Trennstelle sowie
Figur 6 eine Schnittdarstellung einer zweiten Ausführungsvariante einer Trennstelle als Detail der Prinzipdarstellung gemäß Figur 4.

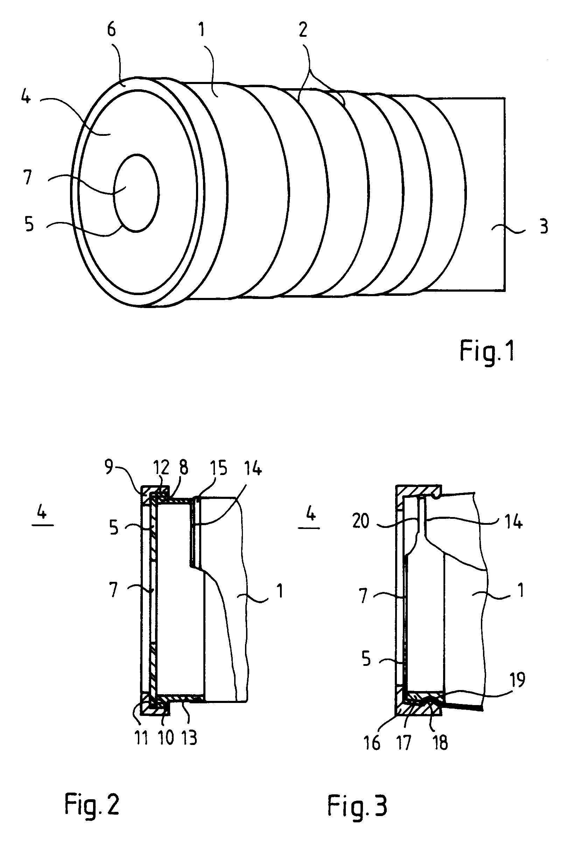

Der in Figur 1 perspektivisch dargestellte Folienüberzug besteht im wesentlichen aus einem Folienschlauch 1 mit teleskopartiger Faltung 2, einer Einstecköffnung 3 und einer Ausgangsöffnung 4. Die Ausgangsöffnung 4 weist eine elastische Membran 5 auf, die in einem ringförmigen Rahmen 6 eingespannt ist. Zum Durchtritt des distalen Endes eines zu umhüllenden chirurgischen Instrumentes, beispielsweise eines Endoskops mit Kamera, ist die Membran 5 mit einer Durchbrechung 7 versehen. Diese schmiegt sich aufgrund ihrer Elastizität dicht an die Außenkonturen des hindurchragenden Instrumententeiles an, so daß ein sicherer Schutz des umhüllten Gerätes vor jeglicher Verschmutzung bzw. Kontamination gewährleistet ist.

Figur 2 zeigt eine erste Variante einer Ausgangsöffnung 4 als Schnittdarstellung. Danach besteht der Rahmen 6 aus einem inneren und einem äußeren Gewindering 8 und 9, wobei der äußere Gewindering 9 als Winkelprofilring ausgebildet ist. Die Membran 5 ist zwischen einer etwas verbreiterten Stirnfläche 10 des inneren Gewinderinges 8 und einer Innenfläche 11 des Winkelprofilringes 9 fest eingeklemmt. Die Klemmung wird durch die Verschraubung der beiden Gewinde 12 aufweisenden Ringe 8 und 9 erreicht.

Der innere Gewindering 8 ist mit einem ringförmigen, aus schweißbarem Material bestehenden Ansatz 13 versehen, welcher mit dem Rand 14 des Folienschlauches 1 durch eine geschlossene Schweißnaht 15 verbunden ist.

Eine weitere Ausführungsform einer Ausgangsöffnung 4 ist in Figur 3 wiedergegeben. Der Rahmen 6 besteht wiederum aus zwei ringförmigen Teilen 16 und 17, welche jedoch nicht durch Verschraubung sondern durch Klemmung miteinander verbunden sind. Das äußere Rahmenteil 16 weist an seiner Innenfläche eine Ringwulst 18 auf, die einer Ringnut 19 an der Außenfläche des inneren Rahmenteiles 17 zugeordnet ist. Die beiden aneinandergrenzenden Flächen sind schwach kegelförmig ausgebildet. Dadurch läßt sich der Rand 14 des Folienschlauches 1 bis über die Ringnut 19 des inneren Rahmenteiles 17 hinüberstreifen und beim Festdrükken des äußeren Rahmenteiles 16 zwischen der Wulst 18 und der Nut 19 einklemmen, ohne daß die Gefahr besteht, daß der Folienschlauchrand 14 beim Aufschieben des äußeren auf das innere Rahmenteil wieder von dem inneren Rahmenteil 17 heruntergeschoben wird.

Vor dem Verbinden der beiden Rahmenteile 16 und 17 wird der Folienschlauchrand 14 über einen unteren Teil des inneren Rahmenteiles 17 und die Membran 5 über den gegenüberliegenden Stirnbereich des inneren Rahmenteiles 17 hinübergelegt. Der Rand 20 der kreisförmigen Membran 5 ragt dabei geringfügig über die Stirnfläche hinüber, so daß die Membran 5 durch das Festdrücken des äußeren auf das innere Rahmenteil in den Raum zwischen den aneinandergrenzenden Flächen in Richtung zur Ringnut 19 hineingezogen und damit straffer gespannt wird.

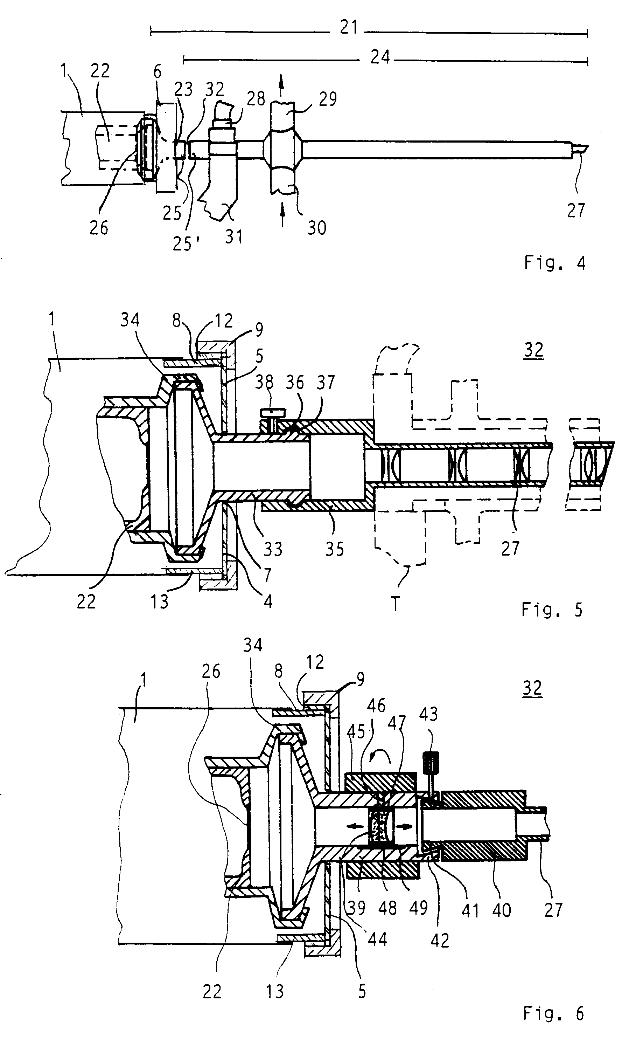

In Figur 4 ist eine Kamera 22 zum Schutz mit einem erfindungsgemäßen Folienüberzug versehen und an einem Arthroskop 21 angeschlossen, welches aus einem kameraseitigen, im wesentlichen zylindrischen Teilstück 23 und einem dem zu arthroskopierenden Gelenk zuzuwendenden Arthroskopteil 24 besteht. Das zylindrische Teilstück 23 ist Teil eines teilbaren Kameraanschlußstücks 25 bzw. 25', welches sowohl der mechanischen Befestigung des Arthroskops 21 an die Kamera 22 als auch der optischen Abbildung des endoskopischen zwischenbildes in die Aufnahmeebene 26 der Kamera 21 dient. Das verbleibende Arthroskopteil 24 weist die eigentlichen Arthroskopbauteile, nämlich einen Optikschaft 27, eine Lichtleitfaser-Beleuchtungseinrichtung 28, eine Absaugvorrichtung 29, eine Spüleinrichtung 30, einen Haltegriff 31 und einen Teil des Kameraanschlußstücks 25 bzw. 25' auf. Zwischen dem kurzen kameraseitigen Teilstück 23 des Kameraanschlußstücks 25 bzw. 25' und dem Arthroskopteil 24 ist eine Trennstelle 32 zum Auswechseln des Arthroskopteiles 24, des Optikschaftes 27 oder auch der Kamera 22 mit dem Teilstück 23 vorgesehen. Das Teilstück 23 weist einen kameraseitigen Mantelbereich zur Befestigung der in einem Rahmen 6 eingespannten elastischen Membran 5 des Folienüberzuges auf. Der Folienüberzug wird mit dem Rahmen 6 über das Teilstück 23 und die Kamera 22 übergestreift, wobei das Teilstück 23, wie aus der Figur 5 ersichtlich, durch die Durchbrechung 7 der Ausgangsöffnung 4 des Folienüberzuges hindurchragt.

Figur 5 zeigt ein Arthroskop gemäß Figur 4 mit einer näher dargestellten ersten Ausführungsvariante der Trennstelle 32. Die elastische Membran 5 der Ausgangsöffnung 4 des Folienüberzuges, die in einem ringförmigen Rahmen 6 eingespannt ist, ist mit einer Durchbrechung 7 versehen, die sich aufgrund ihrer Elastizität dicht an die Außenkonturen des modifizierten Teilstückes 33 anschmiegt und befestigt ist. Das Teilstück 33 ist über eine Klemmhülse 34 mit der Kamera 22 verbunden und gegenüberliegend mit einem im wesentlichen hohlzylindrischen Ansetzstutzen 35 des Optikschaftes 27 verbunden. Diese als Klemmverbindung ausgebildete Trennstelle basiert auf einer Ringwulst/Ringnut-Rastung. Das Teilstück 33 ist an seiner Außenseite mit einer Ringwulst 36 versehen, während der Ansetzstutzen 35 innenseitig eine passende Ringnut 37 aufweist. Um das Einrasten der Ringnut 37 in die Ringwulst 36 zu erleichtern, kann der Ansetzstutzen in Längsrichtung geschlitzt sein. Dadurch wird eine Federwirkung erzielt, die ein weiteres Aufspreizen des Ansetzstutzens 35 zur Überwindung einer entsprechend ausgeprägteren Ringwulst 36 und damit einen besseren Halt ermöglicht. Eine Klemmschraube 38, die der Ringnut 37 benachbart durch den Ansetzstutzen 35 hindurchschraubbar und gegen die Außenkonturen des Teilstücks 33 festziehbar ist, dient der Fixierung der Drehlage zwischen dem Ansetzstutzen 35 und dem Teilstück 33 und damit zwischen der Kamera 22 und einer Optikbaugruppe 27. Die eine Schrägblickoptik aufweisende Optikbaugruppe 27 ist innerhalb einer die Beleuchtungseinrichtung und die Saug/Spül-Kanäle aufnehmenden Trokarhülse T - gestrichelt dargestellt - drehbar gelagert sowie aus dieser herausziehbar. Es ist ersichtlich, daß damit nach dem Öffnen der Trennstelle die Optikbaugruppe leicht zum Wechseln aus der Trokarhülse herausziehbar ist, die an Ort und Stelle verbleiben kann. Das ist insbesondere unter Operationsbedingungen günstig, da somit das Endoskop nicht vollständig neu eingebracht zu werden braucht. Auch die Kamera mit Zuleitung braucht nicht verändert zu werden. Die Auswechselung der Optik erfolgt ausschließlich im sterilen Bereich. Auch die Abgrenzung zum nicht-sterilen Kamerabereich bleibt unangetastet. Die sich mitdrehende Klemmschraube 38 gestattet zusätzlich ein vereinfachtes Wiederauffinden eines blickrichtungs- und damit drehwinkelabhängigen Objektdetails. Ein Objektivwechsel läßt sich somit mit wenigen Handgriffen schnell bewerkstelligen, was insbesondere bei Operationen von wesentlicher Bedeutung ist.

Eine weitere Ausführungsvariante der Trennstelle 32 ist in Figur 6 dargestellt. Der Optikschaft 27 ist wiederum über eine Schnellwechseleinrichtung mit einem entsprechend ausgestalteten Teilstück 39 verbunden. Diese Schnellwechseleinrichtung ist als Ringschwalbenverbindung ausgebildet, wobei ein Ansetzstutzen 40 des Optikschaftes 27 das Einsatzteil 41 und das Teilstück 39 das Aufnahmeteil 42 der Ringschwalbe aufweisen. Der Ansetzstutzen 40 wird mit dem Einsatz 41 in die Ringschwalbenaufnahme 42 des Teilstücks 39 eingehebelt und mittels einer Rändelschraube 43 in der gewünschten Drehlage arretiert.

Das Teilstück 39 ist auch Träger eines fokussierbaren Projektivlinsensystems 44. Ein für die manuelle Verstellung vorgesehener Rändelring 45 ist auf dem Teilstück 39 drehbar gelagert. Mit dem Rändelring 45 ist ein durch die Wandung des Teilstückes 39 hindurchragender Stift 46 verbunden, der in eine schraubenlinienförmig umlaufende Führungsrille der das Linsensystem 44 aufnehmenden Fassung 47 eingreift. Die translatorische Bewegung des Linsensystems 44 wird durch eine zwangsführung der Fassung 47 erreicht, indem ein mit der Fassung 47 verbundener Ansatz 48 in eine Längsnut 49 in der Innenseite des Teilstücks 39 eingreift.

## Patentansprüche

1. Folienüberzug zum Schutz eines chirurgischen Instrumentes, insbesondere eines Endoskops, vor Verunreinigung, aufweisend einen Folienschlauch (1), der durch eine Einstecköffnung (3) und eine dieser gegenüberliegenden Ausgangsöffnung (4) begrenzt ist, wobei die Ausgangsöffnung (4) eine mit einer Durchbrechung (7) versehene elastische Membran (5) aufweist, deren äußerer Rand in einem den Folienschlauch (1) aufspreizenden Rahmen (6) eingespannt ist, wobei
die Membran (5) mit der Durchbrechung (7) sich aufgrund ihrer Elastizität dicht an die Außenkonturen des hindurchragenden Instrumententeiles anschmiegt, so daß ein sicherer Schutz des umhüllten Gerätes vor jeglicher Verschmutzung bzw. Kontamination gewährleistet ist.

2. Folienüberzug nach Anspruch 1, **dadurch gekennzeichnet,** daß die Membran (5) kreisförmig ausgebildet ist.

3. Folienüberzug nach einem der vorangehenden Ansprüche, **dadurch gekennzeichnet,** daß die Membran (5) eine zentrische, kreisförmige Durchbrechung (7) aufweist.

4. Folienüberzug nach einem der vorangehenden Ansprüche, **dadurch gekennzeichnet,** daß der Rahmen (6) Mittel zum Aufspreizen eines Folienschlauchrandes (14) aufweist.

5. Folienüberzug nach Anspruch 4, **dadurch gekennzeichnet,** daß der Rahmen (6) einen ringförmigen Ansatz (13) aus schweißbarem Material aufweist, welcher mit dem Folienschlauchrand (14) verschweißt ist.

6. Folienüberzug nach Anspruch 5, **dadurch gekennzeichnet,** daß der Ansatz (13) aus Weichplastik besteht.

7. Folienüberzug nach einem der vorangehenden Ansprüche, **dadurch gekennzeichnet,** daß der Rahmen (6) aus mehreren Profilringen (8, 9 bzw. 16, 17) besteht, zwischen denen der Folienschlauchrand (14) und/oder die Membran (5) eingespannt sind.

8. Folienüberzug nach Anspruch 6, **dadurch gekennzeichnet,** daß die Profilringe (16, 17) durch Klemmsitz miteinander verbunden sind.

9. Folienüberzug nach Anspruch 8, **dadurch gekennzeichnet,** daß ein innerer, auf seiner Außenseite mit einer Ringnut (19) versehener Profilring (17) und ein äußerer, auf seiner Innenseite mit einer Ringwulst (18) versehener Profilring (16) vorgesehen sind, wobei Ringnut (19) und Ringwulst (18) auch vertauscht sein können.

10. Folienüberzug nach Anspruch 7, **dadurch gekennzeichnet,** daß die Profilringe (8, 9) miteinander verschraubt sind.

11. Folienüberzug nach einem der vorangehenden Ansprüche, **dadurch gekennzeichnet,** daß der Rahmen (6) aus Kunststoff besteht.

12. Folienüberzug nach einem der vorangehenden Ansprüche, **dadurch gekennzeichnet,** daß die Membran (5) aus Gummi, Silikon oder Latex besteht.

## Claims

1. A sheeting cover for the protection from contamination of a surgical instrument, more particularly an endoscope, comprising a tubular sheeting (1) which is delimited by an insertion port (3) and an exit port (4) positioned opposite the insertion port, the exit port (4) having a resilient membrane (5) provided with a perforation (7) whose outer edge is clamped into a frame (6) which spreads the tubular sheeting (1) open, the membrane (5) with the perforation (7), due to its elasticity, closely resting against the outer contours of the part of the instrument projecting through it so that a reliable protection of the enveloped device from any soiling or contamination is ensured.

2. A sheeting cover according to claim 1, characterised in that the membrane (5) is circular.

3. A sheeting cover according to one of the preceding claims, characterised in that the membrane (5) has a central, circular perforation (7).

4. A sheeting cover according to one of the preceding claims, characterised in that the frame (6) has means for spreading a tubular sheeting rim (14) open.

5. A sheeting cover according to claim 4, characterised in that the frame (6) has a ring-shaped attachment (13) made of weldable material which is welded to the tubular sheeting rim (14).

6. A sheeting cover according to claim 5, characterised in that the attachment (13) is made of soft plastic material.

7. A sheeting cover according to one of the preceding claims, characterised in that the frame (6) comprises several profiled rings (8, 9 or 16, 17), between which the tubular sheeting rim (14) and/or the membrane (5) are clamped.

8. A sheeting cover according to claim 6, characterised in that the profiled rings (16, 17) are interconnected by force fit.

9. A sheeting cover according to claim 8, characterised in that it has an inner profiled ring (17) which is provided on its outside with an annular groove (19), and an outer profiled ring (16) which is provided on its inside with an annular seam (18), whilst the annular groove (19) and annular seam (18) may also be interchanged.

10. A sheeting cover according to claim 7, characterised in that the profiled rings (8, 9) may be screwed to one another.

11. A sheeting cover according to one of the preceding claims, characterised in that the frame (6) is made of plastics material.

12. A sheeting cover according to one of the preceding claims, characterised in that the membrane (5) is made of rubber, silicone or latex.

## Revendications

1. Film enveloppant de protection d'un instrument chirurgical, notamment d'un endoscope, contre les impuretés, présentant un tube de film (1) limité par un orifice d'entrée (3) et un orifice de sortie (4) opposé, l'orifice de sortie (4) présentant une membrane (5) élastique dotée d'une découpure (7) et dont le bord externe est pincé dans un cadre (6) déployant le tube de film (1), étant entendu que la membrane (5) avec la découpure (7), par suite de son élasticité, épouse étroitement les contours externes de la partie de l'instrument qui dépasse, de sorte qu'est assurée une protection sûre de l'appareil enveloppé contre toute salissure ou contamination.

2. Film enveloppant selon la revendication 1, caractérisé en ce que la membrane (5) est circulaire.

3. Film enveloppant selon une des revendications précédentes, caractérisé en ce que la membrane (5) présente une découpure (7) centrale circulaire.

4. Film enveloppant selon une des revendications précédentes, caractérisé en ce que le cadre (6) présente des moyens d'écartement d'un bord (14) du tube de film.

5. Film enveloppant selon la revendication 4, caractérisé en ce que le cadre (6) présente un embout annulaire (13) en un matériau soudable, lequel est soudé au bord (14) du tube de film.

6. Film enveloppant selon la revendication 5, caractérisé en ce que l'embout (13) est en une matière plastique souple.

7. Film enveloppant selon une des revendications précédentes, caractérisé en ce que le cadre (6) est composé de plusieurs bagues profilées (8, 9 ou 16, 17) entre lesquelles sont pincés le bord (14) du tube de film et/ou la membrane (5).

8. Film enveloppant selon la revendication 6, caractérisé en ce que les bagues profilées (16, 17) sont raccordées par ajustement serré.

9. Film enveloppant selon la revendication 8, caractérisé en ce que sont prévues une bague profilée interne (17), dotée sur sa face externe d'une gorge annulaire (19) et une bague profilée externe (16), dotée sur sa face interne d'un bourrelet annulaire (18), la gorge annulaire (19) et le bourrelet (18) pouvant également être intervertis.

10. Film enveloppant selon la revendication 7, caractérisé en ce que les bagues profilées (8, 9) sont vissées l'une à l'autre.

11. Film enveloppant selon une des revendications précédentes, caractérisé en ce que le cadre (6) est en matière plastique.

12. Film enveloppant selon une des revendications précédentes, caractérisé en ce que la membrane (5) est en caoutchouc, silicone ou latex.
